# EUROPEAN PATENT SPECIFICATION

(11) **EP 3 047 817 B1**
(45) Date of publication and mention of the grant of the patent: **12.12.2018**
(21) Application number: 16150058.2
(22) Date of filing: 04.01.2016
(51) Int. Cl.: A61C 5/77, A61C 13/00, G06F 19/00, A61C 19/04

(54) **MISSING TOOTH FORM DETERMINATION COMPUTER IMPLEMENTED METHOD AND CORRESPONDING PROGRAM AND COMPUTER READABLE DATA**
COMPUTERIMPLEMENTIERTES VERFAHREN ZUR BESTIMMUNG DER FORM EINES FEHLENDEN ZAHNS UND ENTSPRECHENDES PROGRAMM UND COMPUTERLESBARE DATEN
PROCÉDÉ DE DÉTERMINATION DE FORME DE DENT MANQUANTE PAR ORIDNATEUR ET SON PROGRAMME CORRESPONDANT ET DONNÉES LISIBLES PAR ORIDINATEUR

(30) Priority: 22.01.2015 JP 2015010737
(43) Date of publication of application: 27.07.2016
(73) Proprietor: Shofu Inc., Kyoto 605-0983 (JP)
(72) Inventor: KADOBAYASHI, Yusei, KYOTO, 605-0983 (JP); SHIGEZAWA, Masako, KYOTO, 605-0983 (JP)
(74) Representative: Eisenführ Speiser

(56) References cited:
- JP-A- 2010 017 467
- US-A1- 2004 185 422
- US-A1- 2010 076 581
- US-A1- 2010 145 898
- US-A1- 2013 282 351
- US-A1- 2014 278 278

## Description

### BACKGROUND OF THE INVENTION

### Field of the Invention

The present invention relates to a method of determining a tooth form of a missing tooth to be used in dental treatment, and a program thereof. In particular, the present invention relates to a method of determining a tooth form in the case of producing a crown form using CADCAM software, and a program thereof.

### Description of the Related Art

Conventionally, in the case of restoring the form of a missing tooth, restoration has been performed with reference to the facial features and the forms of adjacent teeth. However, even referring to the facial features and the forms of adjacent teeth, it requires skill to produce a crown which goes well with adjacent teeth from limited information. Further, as a tooth is restored using a CADCAM system nowadays, tooth form data in digital data is demanded.

In recent years, when a tooth is restored using a computer, a crown form to be restored is selected from several types of crown forms prepared in advance, for example. However, there is a difference in form from adjacent teeth by only selecting from limited several types of crown forms.

Japanese Unexamined Patent Application Publication (Translation of PCT Application) No. 2011-521767 introduces production of a prosthesis using a digital image of a patient. However, as a defect site of a patient does not exist, a tooth form of a defect site cannot be determined.

US2013/0282351 A1 describes a method of determining virtual tooth restorations on the basis of scan data of oral structures for use in dental CAD/CAM systems.

### SUMMARY OF THE INVENTION

A method of easily producing a crown form which goes well with adjacent teeth, while using digital data capable of being used in CADCAM even by an unskilled person, has been required. Further, a method of easily producing crown data of a defect site of a patient has also been required.

Respective sites of a living organism are affected by each other at a certain level and are in forms having a uniform tendency in many cases, if they are of the same individual. Even in the case of crown forms, teeth forms of different sites in the same individual are affected by each other at a certain level and are in forms having a uniform tendency. As such, it has been found that it is possible to restore the form of a defect site which goes well with adjacent teeth by selecting an individual having similar crown forms and selecting a crown form of the same site as the defect site from the selected individual.

The present invention is based on this knowledge. Specifically, a missing tooth form determination method of the present invention includes two steps, that is, a tooth form database construction step of constructing a database of a plurality of units of tooth form data, and a missing tooth form searching step of searching the database for a tooth form which is most similar to remaining tooth form data.

In the tooth form database construction step, a plurality of units of tooth form data are acquired using steps including a dentition form taking step of measuring solid forms of at least two teeth and acquiring dentition form data; a tooth form separation step of separating the dentition form data, acquired in the dentition form taking step, for each of the teeth to thereby allow each unit of the dentition form data to be a unit of tooth form data, and grouping units of the tooth form data into a group for the dentition; and a tooth form classification step of classifying the units of the tooth form data, separated in the tooth form separation step, by the tooth type. The units of tooth form data are accumulated, whereby they are put into a database.

The missing tooth form searching step is a step of selecting a form of a missing tooth through the following steps: that is, a remaining tooth form taking step of measuring a remaining tooth of the patient having the missing tooth, and acquiring remaining tooth form data; a tooth form searching step of searching the database for a tooth form which is most similar to the remaining tooth form data; and a missing tooth form selection step of selecting form data of the missing tooth from the group of the units of the tooth form data searched in the tooth form searching step.

A missing tooth form determination program of the present invention is a program for determining a tooth form of a missing tooth of a patient. The program realizes, in a computer, dentition form taking means for measuring solid forms of at least two teeth and acquiring dentition form data; tooth form separation means for separating the dentition form data, acquired by the dentition form taking means, for each of the teeth to thereby allow each unit of the dentition form data to be a unit of tooth form data, and grouping units of the tooth form data; tooth form classification means for classifying the units of the tooth form data, separated by the tooth form separation means, by the tooth type; tooth form database construction means for constructing a database including the units of the tooth form data with use of the dentition form taking means, the tooth form separation means, and the tooth form classification means; remaining tooth form taking means for measuring a form of a remaining tooth of the patient having the missing tooth, and acquiring remaining tooth form data; tooth form searching means for searching the database for a tooth form which is most similar to the remaining tooth form data; and missing tooth form selection means for selecting a form of the missing tooth from the group of the units of the tooth form data searched by the tooth form searching means.

The tooth form separation step preferably includes a mesial and/or distal data addition step of adding and/or correcting data in a mesial direction and/or a distal direction of the separated tooth form data to thereby obtain a crown form.

It is preferable that in the tooth form searching step, the remaining tooth form data is acquired from a tooth adjacent to the missing tooth.

It is also preferable that in the tooth form classification step, the tooth forms are classified into at least fourteen types including central incisors, lateral incisors, canines, first premolars, second premolars, first molars, and second molars, of an upper jaw and a lower jaw respectively, and units of form data of the right and left sides are classified by being inversed.

The form of a defect site can be restored easily from a database including tooth forms.

### BRIEF DESCRIPTION OF THE DRAWINGS

Figure 1 is a schematic diagram in the case where the present invention is carried out over the Internet; and
Figure 2 is a schematic diagram of a tooth form data list.

### DETAILED DESCRIPTION OF THE PREFERRED EMBODIMENT

In the present invention, a patient of whom the tooth form of a missing tooth can be selected is a patient having a remaining tooth. If there is no remaining tooth, it is impossible to extract a tooth form of a missing tooth from a database.

While the present invention is useful even in the case of a missing tooth in which a portion of the crown is missing, the present invention is most effective in the case where the entire crown of a tooth is missing. Even if a plurality of teeth are missing, if there is any remaining tooth, searching can be made based on the remaining tooth.

### <Dentition Form Taking Step>

At least two teeth are teeth taken from the same person. One tooth of them is a tooth for searching for a similar tooth form in a tooth form searching step, and the other tooth is a tooth which is selected as a missing tooth for which a tooth form is acquired. A solid form of a tooth is preferably a crown form.

Dentition form data includes at least two units of tooth form data. If it includes two or more units of tooth form data acquired from the same person, there is no problem even if the two or more teeth are not in a row particularly. The at least two teeth are preferably adjacent teeth, and it is more preferable that they are tooth forms of twenty-eight teeth.

Dentition form data can be acquired by a typical method without any problems.

### <Dentition Form Separation Step>

A method of separating dentition form data for each tooth is to separate dentition form data into units of tooth form data of respective crowns of teeth, specifically. The separated tooth form data of each tooth constitutes a minimum unit of data when extracting a tooth form. It should be noted that if the separated tooth form data of each tooth includes data of gingiva or the like, data of gingiva or the like must be excluded. Further, it is necessary to associate units of tooth form data of respective teeth, separated from dentition form data acquired from the same person, with each other as those belonging to the same group. This is because when selecting tooth form data of a missing tooth, even if extraction is performed using tooth form data (remaining tooth form data) of a remaining tooth of a patient having the missing tooth, tooth form data of the missing tooth cannot be selected unless they are associated as those belonging to the same group.

### <Tooth Form Classification Step>

By classifying units of tooth form data by the tooth type, a site corresponding to the missing tooth in the dentition can be specified. Further, when extracting tooth form data, by specifying the site of the searching target, it is possible to reduce a burden of searching. If the types of the remaining tooth form data used for searching and the tooth form data searched in the database differ from each other, the similarity is low even if a tooth form of the same type as that of the missing tooth is selected.

### <Tooth Form Database Construction Step>

In a tooth form database construction step, it is important to construct a database of a plurality of units of tooth form data, and it is important to collect units of dentition form data from many clients who are providers of data such as dentists, dental technicians, university professors, and dental hygienists through Internet lines or the like. For example, the present invention can be realized on the Internet. In that case, it is preferable to ask a client, requesting data of a tooth form, for providing dentition form data of a remaining tooth and the like of a patient requesting data of a tooth form. In this way, units of tooth form data stored in the database are increased, whereby it is possible to obtain tooth form data having higher similarity to the form of the target missing tooth. It should be noted that besides dentition form data, tooth form data may be collected, for example.

### <Remaining Tooth Form Taking Step>

Next, a remaining tooth form taking step will be described.

A method of measuring a crown form of a remaining tooth of a patient having a missing tooth can be performed by a typical method with no problems. For example, it can be acquired as remaining dentition form data.

It is desirable to separate the acquired remaining dentition form data for each tooth. Specifically, the remaining dentition form data is separated into a unit of remaining tooth form data of each crown of a remaining tooth, and the separated tooth form data of each remaining tooth constitutes a minimum unit of data when extracting a tooth form. It should be noted that if the separated tooth form data of each tooth includes data of gingiva or the like, data of gingiva or the like must be excluded.

### <Tooth Form Searching Step>

From the units of tooth form data in the database, tooth form data of a tooth form which is most similar to the remaining tooth form data is searched. A method of determining the similarity of data is not limited, particularly. However, it is preferable to check the similarity by handing the relationship between units of tooth form data as the same tooth form data.

In order to perform searching in the database efficiently, it is preferable to perform searching from a set of groups only including data of tooth forms of the same type as that of the target missing tooth and the remaining tooth form data.

If there are a plurality of remaining teeth, it is possible to perform selection by using units of tooth form data of respective remaining teeth, in which selection can be made based on the fact that a plurality of units of tooth form data are similar.

### <Missing Tooth Form Selection Step>

Next, by selecting tooth form data of a tooth of the same type as that of the target missing tooth from a group of most similar tooth form data, a form of the missing tooth is selected. It is preferable to select a tooth form of the same site as that of the missing tooth.

Further, it is preferable to have a mesial and/or distal data addition step of adding and correcting data in a mesial and/or distal direction of a tooth form with respect to the separated tooth form data. In the case of an adjacent tooth, as there is an adjacent tooth in a mesial or distal direction, data in the crown cannot be acquired. As such, in the case where mesial and distal data is constructed, it is preferable to form a plane from an end portion of a lingual surface to an end portion of a labial surface smoothly. This is because when selection is made as a form of the missing tooth, data of a crown part is required.

Remaining tooth form data is preferably tooth form data of a tooth adjacent to the missing tooth. In that case, as the remaining tooth form data also has data of a surface shape of the missing tooth side, it is suitable for searching for and selecting tooth form data of a most similar tooth. Further, a tooth adjacent to the target missing is most suitable for determining the similarity. Further, it is preferable to search for one or more units of remaining tooth form data of a most similar tooth form, among a central incisor, a canine, a first premolar, a second premolar, a first molar, and a second molar which have features in the crown shapes.

In the tooth form classification step, it is preferable to classify the units of tooth form data into tooth types of fourteen sites such as central incisors, lateral incisors, canines, first premolars, second premolars, first molars, and second molars, of the upper jaw and the lower jaw, respectively. It is also possible to classify units of tooth form data into tooth types of sixteen sites by adding third molars. Further, it is possible to classify them into tooth types of twenty-four sites by adding deciduous central incisors, deciduous lateral incisors, deciduous canines, first deciduous molars, and second deciduous molars of the respective upper and lower jaws, or of twenty-eight sites.

Further, the tooth form data at bilaterally symmetric positions can be classified by inverting them each other. It should be noted that symmetric positions of the left dentition and the right dentition may be uniformed in the database. Further, in the case of searching for a tooth in one of the left dentition and the right dentition, data at the symmetric position in the other one of the left dentition and the right dentition may be searched.

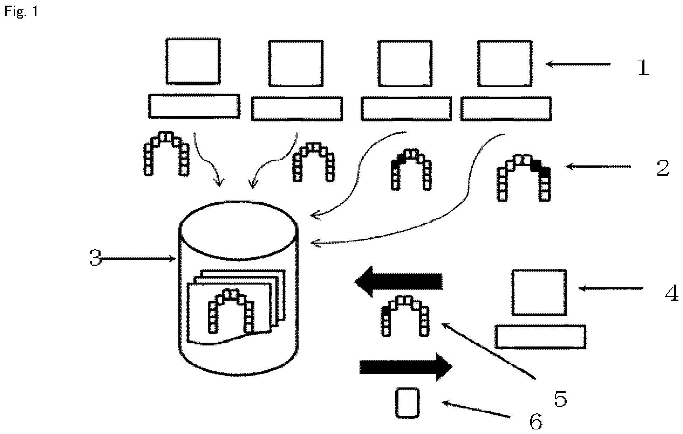

Next, a missing tooth form determination system of the present invention for performing a missing tooth form determination method of the present invention will be described using Figure 1.

Figure 1 shows a schematic diagram of the case of performing a system of the present invention over the Internet.

It is assumed that a personal computer 1 as a client terminal connected with an Internet line is provided at a place of a client such as a dental technician, a dentist, or the like, for example. The personal computer 1 is one having a well-known configuration including a memory unit, a display unit, an input unit, a processing unit, a communication unit, and the like. By a missing tooth form determination program of the present invention, a dentition form taking means which performs the dentition form taking step, described above, is realized inside thereof. Dentition form data taken from a patient is transmitted to a database server through the Internet line and stored in a database 3, by the personal computer 1.

In the present embodiment, the database server is one having a well-known configuration including a memory unit, a display unit, an input unit, a processing unit, a communication unit, and the like. By the missing tooth form determination program of the present invention, a tooth form separation means, a tooth form classification means, and a tooth form database construction means, which perform the tooth form separation step, the tooth form classification step, and the tooth form database construction step described above, respectively, are realized inside thereof. It should be noted that in Figure 1, while the database server connected with the personal computer 1 through the Internet line includes the database 3, the personal computer 1 may include the database 3 therein.

The tooth form separation means separates dentition form data, transmitted from the personal computer 1, for each tooth in such a manner as not to break the crown forms, to thereby constitute units of tooth form data. The tooth form separation means manages units of tooth form data of the teeth of the same dentition while associating with each other as those in the same group. The tooth form separation means also manages units of tooth form data of the teeth of the dentitions of the upper and lower jaws of the same person while associating with each other as those in the same group. In the example of Figure 1, the tooth form separation means manages them as a group A, a group B, a group C, and the like.

The tooth form classification means classifies units of tooth form data of respective teeth by the tooth type. Specifically, the tooth form classification means of the present embodiment classifies the units of tooth form data into central incisors, lateral incisors, canines, first premolars, second premolars, first molars, second molars, deciduous central incisors, deciduous lateral incisors, deciduous canines, first deciduous molars, and second deciduous molars, of the upper jaw and the lower jaw, respectively. Some teeth may be missing, depending on dentition form data. In that case, the fact that some teeth are missing is registered in the database.

Particularly, in the present embodiment, the missing tooth form determination program of the present invention realizes, in the database server, a data addition and/or correction means for adding and/or correcting data in such a manner that the form of a tooth specified by the separated tooth form data becomes a crown form. Particularly, in the present embodiment, a mesial and/or distal data addition and/or correction means for adding and/or correcting data in mesial and/or distal directions to obtain a crown form is realized in the database server. Data is added and/or corrected so as to obtain a crown form in which the surface of a tooth form moves smoothly.

The tooth form database construction means constitutes the database 3 from a plurality of units of tooth form data acquired by using the dentition form taking means, the tooth form separation means, and the tooth form classification means. Specifically, the database 3 is configured as a tooth form database in which the acquired units of tooth form data are managed as a group of dentitions of the same person, and are managed while being classified into the tooth types, as described above.

It is preferable that remaining tooth form data selected in a missing tooth form selection step performed by a missing tooth form selection means, described below, is added to the tooth form database from time to time, and that the data stored in the database is updated.

As shown in Figure 1, a personal computer 4 as a client terminal connected with an Internet line is provided at a place of a client such as a dental technician or a dentist, for example. In the present embodiment, the personal computer 1 and the personal computer 4 are provided at different places. It should be noted that the personal computer 1 and the personal computer 4 may be provided at the same place, and the means realized by the personal computer 4, described below, may be realized in the personal computer 1. In the personal computer 4, a remaining tooth form acquisition means, a tooth form searching means, and a missing tooth selection means, which respectively perform the remaining tooth form acquisition step, the tooth form searching step, and the missing tooth form selection step, described above, are realized inside thereof, by the missing tooth form determination program of the present invention.

The remaining tooth form acquisition means acquires remaining tooth form data 5, based on the result of measuring a crown form of a remaining tooth of the patient having a missing tooth. By the personal computer 4, the remaining tooth form data 5 is transmitted to the database server and is stored in the database 3.

The tooth form search means searches the database 3, constructed by the tooth form database construction means, for tooth form data which is most similar to the remaining tooth form data 5. A search target is tooth form data of the same classification as that of the remaining tooth form data, among the units of data in the database. Further, searching may be performed on grouped units of tooth form data, which will be described below.

The missing tooth form selection means specifies the most similar tooth form data searched, and specifies a group to which the specified tooth form data belongs. By selecting tooth form data of a tooth of the same type as that of the target missing tooth from the specified group, a form 6 of the missing tooth is selected and the selected form 6 of the missing tooth is transmitted to the personal computer 4.

According to the present invention, the client of the personal computer 4 is able to obtain the tooth form data of the form 6 of the missing tooth, by sending the remaining tooth form data 5 taken from the patient.

Here, grouping of units of tooth form data for searching will be described. Grouping may include first grouping and second grouping, for example.

The first grouping will be described.

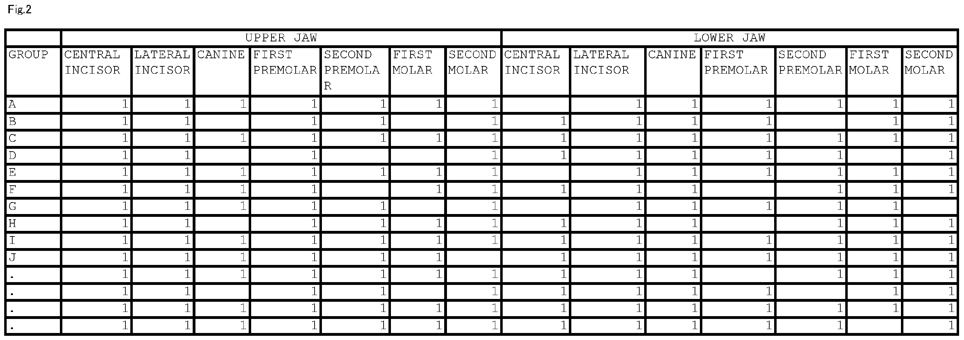

In the tooth form searching step, the tooth form searching means searches for tooth form data which is most similar to remaining tooth form data, and in the missing tooth form selection step, the missing tooth form selection means selects a form (data) of a missing tooth from a group to which the most similar tooth form data belongs. As such, it is a condition for the tooth form searching means to perform searching on groups which include both the data (missing tooth form data) of the teeth of the same classification as that of the target missing tooth and the remaining tooth form data. As such, for each group, it is preferable to prepare a tooth form data list showing presence or absence of tooth form data of respective classifications in advance, so as to cause the data in the database 3 on which searching is performed to be only data of groups having both the data (missing tooth form data) of the teeth in the same classification as that of the target missing tooth and the remaining tooth form data in the tooth form searching step. Figure 2 shows a schematic diagram of the tooth form data list.

In other words, in the first grouping, by preparing, in advance, a tooth form data list showing presence or absence of tooth form data of respective classifications regarding the groups of data included in the database 3, it is possible to extract only groups having the missing tooth form data and the remaining tooth form data. The groups extracted through the first grouping can be the target on which searching is performed by the tooth form searching means.

Next, second grouping will be described.

By classifying the units of tooth form data according to the types of teeth, and setting only tooth data of a particular classification to be a searching target, the number of units of tooth form data to be searched is decreased. In the second grouping, groups are extracted by expressing form features of the tooth form data in numerical values and classifying them according to the relations shown by the numerical values. The groups extracted in the second grouping can be set as targets on which searching is performed by the tooth form searching means. Particularly, it is preferable to express feature points in numerical values, and classify them by the relations shown by the ratios of the numerical values.

Regarding the second grouping, grouping may be performed in advance or performed using the remaining tooth form data to be used for searching.

First, the case of performing grouping in advance will be described.

Regarding the respective units of tooth form data, feature points are measured, a ratio value between a measurement result of one feature point and a measurement result of another feature point is made into data, and setting a plurality of certain numerical ranges by the types of data of ratio values, whereby the units of tooth form data can be classified according to the data of the ratio values.

Here, in order to prevent search omission, it is preferable that numerical ranges of the respective types of data of ratio values overlap with each other. For example, in the case of classifying into ranges of ratios of 1:1 to 1:3 and 1:3 to 1:5, numerical ranges are set so as to overlap with each other partially like 1:1 to 1:3.5 and 1:3 to 1:5. In that case, the tooth form data in the overlapped range may be classified into both classifications. In that case, it is preferable that proportion of the tooth form data in the overlapped range should be 3% to 20% for each type of data of ratio value.

Next, the case of performing grouping using the remaining tooth form data to be used for searching will be described.

Regarding the respective units of tooth form data, feature points are measured, a ratio value between a measurement result of one feature point and a measurement result of another feature point is made into data, and a tooth feature point data list is created, in which data of a ratio value is associated with each unit of tooth form data.

In that case, the tooth form searching means also measures feature points similarly regarding the remaining tooth form data, and obtains data of ratio values. Then, the tooth form searching means sets numerical ranges including ratio values of the remaining tooth form data for each type of data of ratio values. Then, by collating the set numerical ranges with the ratio values of the tooth form data in the tooth feature point data list, only tooth form data in which the ratio values are within the set numerical range is classified as the tooth form data to be searched.

As specific classification, for example, regarding a first molar, it is possible to classify it by showing a distance relation between fossae in a ratio and expressing it in a numerical value, and expressing the positional relation of the fossae in an angle.

While description has been given on the case of including a plurality of personal computers and a database server in the embodiment described above, the present invention may be carried out by only personal computers by allowing one personal computer to function as a server, or may be carried out by single personal computer.

The present invention can be comprehended as a storage medium which stores the missing tooth form determination program of the present invention.

Further, the present invention can also be comprehended as a device which executes the missing tooth form determination method or the missing tooth form determination program of the present invention.

Further, the present invention can also be comprehended as a system which executes the missing tooth form determination method or the missing tooth form determination program of the present invention.

The present invention has industrial applicability because a missing tooth of a patient is able to be produced.

### Reference Signs List

- 1: personal computer
- 2: dentition form data
- 3: database
- 4: personal computer
- 5: remaining tooth form data
- 6: missing tooth form data

## Claims

1. A missing tooth form determination computer implemented method for determining a tooth form of a missing tooth of a patient, the method comprising:
a dentition form taking step of measuring solid forms of at least two teeth and acquiring dentition form data (2);
a tooth form separation step of separating the dentition form data (2), acquired in the dentition form taking step, for each of the teeth to thereby allow each unit of the dentition form data (2) to be a unit of tooth form data, and grouping units of the tooth form data into a group for the dentition;
a tooth form classification step of classifying the units of the tooth form data, separated in the tooth form separation step, by a tooth type;
a tooth form database construction step of constructing a database (3) including the units of the tooth form data with use of the dentition form taking step, the tooth form separation step, and the tooth form classification step;
a remaining tooth form taking step of measuring a crown form of a remaining tooth of the patient having the missing tooth, and acquiring remaining tooth form data (5);
a tooth form searching step of searching the database (3) for a tooth form which is most similar to the remaining tooth form data (5); and
a missing tooth form selection step of selecting form data of the missing tooth from the group for the dentition to which the units of the tooth form data searched in the tooth form searching step belong to.

2. A missing tooth form determination program for performing the computer implemented method according to any of claims 1 and 3 to 5, the program realizing, in a computer (1, 4):
dentition form taking means for measuring solid forms of at least two teeth and acquiring dentition form data (2);
tooth form separation means for separating the dentition form data (2), acquired by the dentition form taking means, for each of the teeth to thereby allow each unit of the dentition form data (2) to be a unit of tooth form data, and grouping units of the tooth form data into a group for the dentition;
tooth form classification means for classifying the units of the tooth form data, separated by the tooth form separation means, by a tooth type;
tooth form database construction means for constructing a database (3) including the units of the tooth form data with use of the dentition form taking means, the tooth form separation means, and the tooth form classification means;
remaining tooth form taking means for measuring a form of a remaining tooth of the patient having the missing tooth, and acquiring remaining tooth form data (5);
tooth form searching means for searching the database (3) for a tooth form which is most similar to the remaining tooth form data (5); and
missing tooth form selection means for selecting a form of the missing tooth from the group for the dentition to which the units of the tooth form data searched by the tooth form searching means belong to.

3. The missing tooth form determination method according to claim 1, wherein
the tooth form separation step includes a mesial and/or distal data addition step of adding and/or correcting data in a mesial direction and/or a distal direction of the separated tooth form data to thereby obtain a crown form.

4. The missing tooth form determination method according to claim 1 or 3, wherein
in the tooth form searching step, the remaining tooth form data (5) is acquired from a tooth adjacent to the missing tooth.

5. The missing tooth form determination method according to any one of claims 1, 3, and 4, wherein
in the tooth form classification step, the tooth forms are classified into at least fourteen types including central incisors, lateral incisors, canines, first premolars, second premolars, first molars, and second molars of an upper jaw and a lower jaw respectively, and units of form data of right and left sides are classified by being inversed.

6. A storage medium that stores the missing tooth form determination program according to claim 2.

## Patentansprüche

1. Computerimplementiertes Verfahren zur Bestimmung der Form eines fehlenden Zahnes zum Bestimmen einer Form eines Zahnes eines fehlenden Zahnes eines Patienten, wobei das Verfahren umfasst:
einen Schritt des Gebissformnehmens, bei dem feste Formen von mindestens zwei Zähnen gemessen werden und Gebissformdaten (2) erfasst werden;
einen Zahnformtrennungsschritt des Trennens der Gebissformdaten (2), die in dem Schritt des Gebissformnehmens erfasst wurden, für jeden der Zähne, um dadurch zu ermöglichen, dass jede Einheit der Gebissformdaten (2) eine Einheit von Daten der Form eines Zahnes ist, und des Gruppierens von Einheiten der Daten der Form eines Zahnes in eine Gruppe für das Gebiss;
einen Zahnformklassifikationsschritt zur Klassifizierung der Einheiten der Daten der Form eines Zahnes, die im Zahnformtrennungsschritt voneinander getrennt sind, nach einer Art des Zahnes;
einen Zahnformdatenbank-Konstruktionsschritt des Konstruierens einer Datenbank (3), die die Einheiten der Daten der Form eines Zahnes unter Verwendung des Schritts des Gebissformnehmens, des Zahnformtrennungsschritts und des Zahnformklassifikationsschritts beinhaltet;
einen Schritt des Nehmens der Form eines verbleibenden Zahnes zum Messen der Form einer Krone eines verbleibenden Zahnes des Patienten, der den fehlenden Zahn hat, und Erfassen von Daten der Form eines verbleibenden Zahnes (5);
einen Suchschritt der Form eines Zahnes durch Durchsuchen der Datenbank (3) nach einer Form eines Zahnes, die den Daten der Form des verbleibenden Zahnes (5) am ähnlichsten ist; und
einen Auswahlschritt der Form eines fehlenden Zahnes des Auswählens von Daten der Form des fehlenden Zahnes aus der Gruppe für das Gebiss, zu dem die Einheiten der Daten der Form eines Zahnes, nach denen in dem Suchschritt der Form eines Zahnes gesucht wurde, gehören.

2. Programm zur Bestimmung der Form eines fehlenden Zahnes zum Durchführen des computerimplementierten Verfahrens gemäß einem beliebigen der Ansprüche 1 und 3 bis 5, wobei das Programm in einem Computer (1,4) bewirkt:
Mittel zum Nehmen einer Gebissform, die feste Formen von mindestens zwei Zähnen messen und Gebissformdaten (2) erfassen;
Zahnformtrennungsmittel zum Trennen der Gebissformdaten (2), die von den Mitteln zum Gebissformnehmen erfasst wurden, für jeden der Zähne, um dadurch zu ermöglichen, dass jede Einheit der Gebissformdaten (2) eine Einheit von Daten der Form eines Zahnes ist, und zum Gruppieren von Einheiten der Daten der Form eines Zahnes in eine Gruppe für das Gebiss;
Zahnformklassifikationsmittel zum Klassifizieren der Einheiten der Daten der Form eines Zahnes, die durch die Zahnformtrennungmittel voneinander getrennt sind, nach einer Art des Zahnes;
Zahnformdatenbank-Konstruktionsmittel zum Konstruieren einer Datenbank (3), die die Einheiten der Daten der Form eines Zahnes unter Verwendung der Mittel zum Gebissformnehmen, der Zahnformtrennungsmittel und der Zahnformklassifikationsmittel beinhaltet;
Mittel zum Abnehmen der Form eines verbleibenden Zahnes zum Messen einer Form eines verbleibenden Zahnes des Patienten, der den fehlenden Zahn hat, und zum Erfassen von Daten der Form eines verbleibenden Zahnes (5);
Mittel zur Suche der Form eines Zahnes des Durchsuchens der Datenbank (3) nach einer Form eines Zahnes, die den Daten der Form des verbleibenden Zahnes (5) am ähnlichsten ist; und
Mittel zur Auswahl der Form eines fehlenden Zahnes des Auswählens einer Form des fehlenden Zahnes aus der Gruppe für das Gebiss, zu dem die Einheiten der Daten der Form eines Zahnes, nach denen durch die Mittel zur Suche der Form eines Zahnes gesucht wurde, gehören.

3. Verfahren zur Bestimmung der Form eines fehlenden Zahnes gemäß Anspruch 1, wobei
der Zahnformtrennungsschritt einen mesialen und/oder distalen Daten-Additionsschritt des Addierens und/oder Korrigierens von Daten in einer mesialen Richtung und/oder einer distalen Richtung der getrennten Daten der Form der Zähne umfasst, um dadurch eine Form einer Krone zu erhalten.

4. Verfahren zur Bestimmung der Form eines fehlenden Zahnes gemäß Anspruch 1 oder 3, wobei
in dem Suchschritt der Form eines Zahnes die Daten der Form des verbleibenden Zahnes (5) von einem dem fehlendem Zahn benachbarten Zahn erfasst werden.

5. Verfahren zur Bestimmung der Form eines fehlenden Zahnes gemäß einem beliebigen der Ansprüche 1, 3, und 4, wobei
in dem Zahnformklassifikationsschritt die Formen der Zähne in mindestens vierzehn Typen, umfassend mittlere Schneidezähne, seitliche Schneidezähne, Eckzähne, erste Prämolaren, zweite Prämolaren, erste Mahlzähne, und zweite Mahlzähne eines Oberkiefers bzw. eines Unterkiefers klassifiziert werden, und Einheiten von Daten der Form von rechten und linken Seiten dadurch klassifiziert sind, dass sie invertiert sind.

6. Speichermedium, das das Programm zur Bestimmung der Form eines fehlenden Zahnes gemäß Anspruch 2 speichert.

## Revendications

1. Procédé, implémenté par ordinateur, de détermination de forme de dent manquante, destiné à déterminer une forme de dent d'une dent manquante d'un patient, le procédé comprenant:
une étape de prise de forme de dentition consistant à mesurer des formes solides d'au moins deux dents et à acquérir des données de forme de dentition (2);
une étape de séparation de forme de dent consistant à séparer les données de forme de dentition (2), acquises dans l'étape de prise de forme de dentition, pour chacune des dents afin de permettre ainsi que chaque unité des données de forme de dentition (2) est une unité de données de forme de dent, et à regrouper des unités des données de forme de dent dans un groupe pour la dentition;
une étape de classification de forme de dent consistant à classifier les unités des données de forme de dent, séparées dans l'étape de séparation de forme de dent, par un type de dent;
une étape de construction de base de données de forme de dent consistant à construire une base de données (3) comprenant les unités des données de forme de dent en utilisant l'étape de prise de forme de dentition, l'étape de séparation de forme de dent et l'étape de classification de forme de dent;
une étape de prise de forme de dent restante consistant à mesurer une forme de couronne d'une dent restante du patient ayant la dent manquante et à acquérir des données de forme de dent restante (5);
une étape de recherche de forme de dent consistant à rechercher dans la base de données (3) une forme de dent qui ressemble le plus aux données de forme de dent restante (5); et
une étape de sélection de forme de dent manquante consistant à sélectionner des données de forme de la dent manquante dans le groupe pour la dentition auquel appartiennent les unités des données de forme de dent recherchées dans l'étape de recherche de forme de dent.

2. Programme de détermination de forme de dent manquante pour mettre en oeuvre le procédé implémenté par ordinateur selon l'une quelconque des revendications 1 et 3 à 5, le programme réalisant, dans un ordinateur (1, 4):
des moyens de prise de forme de dentition pour mesurer des formes solides d'au moins deux dents et pour acquérir des données de forme de dentition (2);
des moyens de séparation de forme de dent pour séparer les données de forme de dentition (2) acquises par les moyens de prise de forme de dentition, pour chacune des dents, afin de permettre ainsi que chaque unité des données de forme de dentition (2) est une unité de données de forme de dent, et pour regrouper des unités des données de forme de dent dans un groupe pour la définition;
des moyens de classification de forme de dent pour classifier les unités des données de forme de dent, séparées par les moyens de séparation de forme de dent, par un type de dent;
des moyens de construction de base de données de forme de dent pour construire une base de données (3) comprenant les unités des données de forme de dent en utilisant les moyens de prise de forme de dentition, les moyens de séparation de forme de dent et les moyens de classification de forme de dent;
des moyens de prise de forme de dent restante pour mesurer une forme d'une dent restante du patient ayant la dent manquante et pour acquérir des données de forme de dent restante (5);
des moyens de recherche de forme de dent pour rechercher dans la base de données (3) une forme de dent qui ressemble le plus aux données de forme de dent restante (5); et
des moyens de sélection de forme de dent manquante pour sélectionner une forme de la dent manquante dans le groupe pour la dentition auquel appartiennent les unités des données de forme de dent recherchées par les moyens de recherche de forme de dent.

3. Procédé de détermination de forme de dent manquante selon la revendication 1, dans lequel
l'étape de séparation de forme de dent comprend une étape d'addition de données mésiale et/ou distale consistant à ajouter et/ou à corriger des données dans une direction mésiale et/ou une direction distale des données de forme de dent séparées pour obtenir ainsi une forme de couronne.

4. Procédé de détermination de forme de dent manquante selon la revendication 1 ou 3, dans lequel
dans l'étape de recherche de forme de dent, les données de forme de dent restante (5) sont acquises à partir d'une dent adjacente à la dent manquante.

5. Procédé de détermination de forme de dent manquante selon l'une quelconque des revendications 1, 3 et 4, dans lequel
dans l'étape de classification de forme de dent, les formes de dent sont classifiées en au moins quatorze types, comprenant les incisives centrales, les incisives latérales, les canines, les premières prémolaires, les deuxièmes prémolaires, les premières molaires et les deuxièmes molaires d'une mâchoire supérieure et d'une mâchoire inférieure, respectivement, et des unités de données de forme de côtés droit et gauche sont classifiées en étant inversées.

6. Support de stockage qui stocke le programme de détermination de forme de dent manquante selon la revendication 2.
